# EUROPEAN PATENT APPLICATION

(11) **EP 4 473 949 A1**
(43) Date of publication of application: **11.12.2024**
(21) Application number: 22925138.4
(22) Date of filing: 04.07.2022
(51) Int. Cl.: A61F 6/04, B65D 65/24, B65D 65/28, B65D 67/02

(54) **CONDOM PACKAGE**

(30) Priority: 02.02.2022 RU 2022102436
(71) Applicant: Gerasimenko, Vadim Mihajlovich, Saint-Petersburg, 191186 (RU)
(72) Inventor: Gerasimenko, Vadim Mihajlovich, Saint-Petersburg, 191186 (RU)
(74) Representative: Spengler, Robert
(86) International application number: PCT/RU2022/000212
(87) International publication number: WO 2023/149815

(57) **Abstract**

The invention relates to barrier contraceptives, more particularly, to a packing for a condom, which ensures that the condom is securely held until it is completely unfolded. The packing for a condom comprises a covering with a holder inside, and the holder is equipped with fasteners designed to hold the condom in a folded state and located on the inner and/or outer side of the folded condom, wherein in each fastener and/or between at least one pair consisting of an inner and an outer fastener a gap of variable size is formed, which, in one position, allows the folded condom to be placed in the fasteners through it, and in the other position it does not allow the completely folded condom to separate from the fasteners.

## Description

### TECHNICAL FIELD

The invention relates to barrier contraceptives, more particularly, to a packing for a condom, which ensures that the condom is securely held until it is completely unfolded.

### PRIOR ART

Traditional packing for a condom represents a reliable covering made of flexible sheet material designed to store a condom sealed. For convenient usage such packings are provided with means to open the covering, for example a groove, a site with lower reliability on the edge of the packing, or an opening tape with one end left out to break the covering.

A disadvantage of the traditional packing for a condom is a long and inconvenient packing opening process, because before using the condom one has to waste time looking for a covering opening tool provided with the packing, for example, the groove, and next, having taken the packing edges on both sides with both hands, pulling it apart to make a tear. Under the circumstances when a condom is about to be used, extra seconds wasted to make the abovementioned motions, may be critical for a person. Herein, there is a chance to damage a condom in case of negligent and hurried opening of the packing, because a person in tense emotional state may apply unnecessary extra force to tear the covering. Besides, when using a condom as designed, the contact between fingers and the condom itself is inevitable, thus compromising the hygiene required. The abovementioned disadvantages of the traditional packing for a condom may result in refusal to use the condom.

Known is a packing for a condom (patent EP 1377242 B1, published on May 24th 2006). This packing consists of a covering with a condom inside, and two flat side grippers connected to it and to each other. The covering comprises an opening line, located by its central crosswise perimeter, parallel to side grippers, that, when opened, splits the covering into two similar parts. Herein, the covering is provided with four hook-shaped holders located at the corners of the covering to fix the condom within. The known packing is opened via pushing the side grippers with fingers resulting in tearing the packing apart by the opening line. Then the grippers are pulled apart to split the packing into two similar parts comprising the condom. Next, via catching the side grippers with fingers, the condom is put on the erect penis and removed from the packing via detaching it from the hook-shaped holders.

Known is a packing for a condom [patent RU 2515852, published on May 20th, 2014], consisting of a covering with the condom inside, two side grippers connected hereto and hook-shaped holders located inside the covering to fix the condom in the covering. The covering has an opening line located by its central crosswise perimeter that, when opened, splits the covering into two parts. The covering surface is provided with at least two enforcement ribs located parallel to the opening line and symmetrical about it to act as pressure concentrators during opening the covering via breaking it up. Each side gripper with its one end is put inside the covering to be connected with at least one hook-shaped holder, while at the opposite side that is free of the covering, each side gripper is provided with a rest that fixes fingers position.

A disadvantage of the known technical solutions using hook-shaped holders inside the covering is unreliable holding of a folded condom herein, since the condom can fall out of the holders when opening the covering before unwinding it.

### SUMMARY OF THE INVENTION

The technical result is to provide secure and reliable holding the condom in the holder in order to prevent its premature loss during transportation or storage and when opening the covering, as well as ensuring uniform unwinding and quick separation from the packing holder without fingers contacting the condom after at least partially unwinding it.

The technical result is achieved due to the fact that the packing for a condom comprises a covering with a holder inside, and the holder is equipped with fasteners designed to hold the condom in a folded state and located on the inner and/or outer side of the folded condom, wherein in each fastener and/or between at least one pair consisting of an inner and an outer fastener a gap of variable size is formed.

Embodiments for the main technical solution development are possible, wherein:
- the covering is provided with an opening line;
- the holder consists of at least two parts, and each part has fasteners;
- the holder is provided with side grippers located on two opposite sides of the holder and designed to be held with hand fingers when opening the packing;
- the fastener is made hook-shaped;
- the fastener is designed to be unbent to place the folded condom ring inside and then taking initial geometry;
- the fastener is designed to open to place the folded condom ring inside and then close, taking initial position.

Thus, due to the combination of essential features, it became possible to securely and reliably hold the condom in the holder due to the gap of variable size. Uniform unwinding and quick separation of the condom from the holder without fingers contacting the condom after at least partially unwinding it to a ring size not exceeding the size of the gap is also provided. In addition, it became possible to improve the serviceability of the packing due to the possibility of the required orientation of the product during its practical use, which does not require visual control, ensuring fast and convenient extraction of a condom simultaneously retaining its hygiene.

### BRIEF DESCRIPTION OF THE FIGURES

Examples of particular embodiments of the claimed invention and its practical applicability are disclosed herein with reference to the following description and the drawings.
Fig.1 shows a schematic view for the opened packing for a condom.
Fig.2 shows a condom holder with inner fasteners (lateral view).
Fig.3 shows the condom holder with inner fasteners and a condom placed therein (lateral view).
Fig.4 shows the condom holder with inner and outer fasteners (lateral view).
Fig.5 shows the condom holder with inner and outer fasteners and a condom placed therein (lateral view).

### DETAILED DESCRIPTION OF THE INVENTION

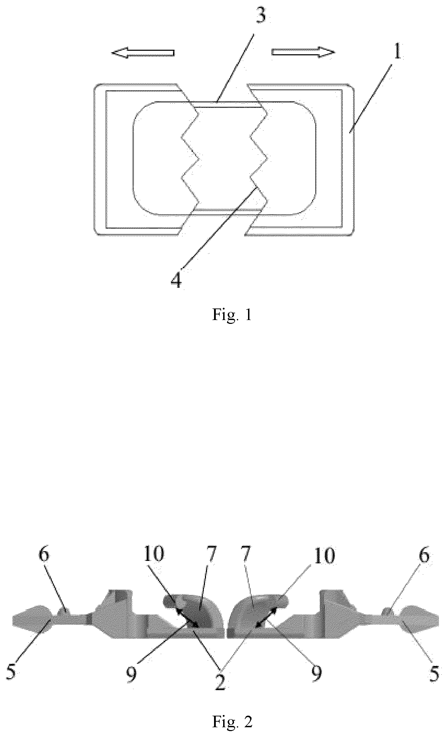

The packing for a condom (Figures 1-5) consists of a covering 1 with a holder 2 of a condom 3 inside in a folded state.

The covering 1 is made of a very touchy and sensitive material and has an opening line 4 (Fig.1), a site where the material has lower reliability compared to the rest of the covering 1. The covering 1 can be provided with other known means to open the covering for example, a groove, an opening tape, etc.

The holder 2 can be made of at least two parts connected to each other by a temporary connection (for example, glued or connected by jumpers), collapsing when the package is stretched in different directions, or connected via a condom 3 located therein and separating from each other when extracting the condom 3. The parts of the holder 2 are held relative to each other, including by the covering 1.

The middle part of the holder 2 has a hole (not shown on drawing), along the perimeter of which the condom 3 ring is placed for convenient use of the condom 3 as designed.

The holder 2 may be provided with side grippers 5 located on two opposite sides of the holder 2 and designed to be held with hand fingers when opening the covering 1. The side grippers 5 may be provided with finger rests 6 (or ridging) to prevent fingers from sliding off when opening the covering 1. The side grippers 5 can also be made curved (not shown on drawing) to create a larger arm of force when opening the covering 1.

The holder 2 can be made of a flexible or rigid material, for example, a polymer material by casting or three-dimensional printing.

The holder 2 can be entirely placed into the covering 1. It is also possible for the side grippers 5 to at least partially protrude from the covering 1. In this case, the covering is leak-proof glued or soldered throughout the perimeter of the side grippers 5.

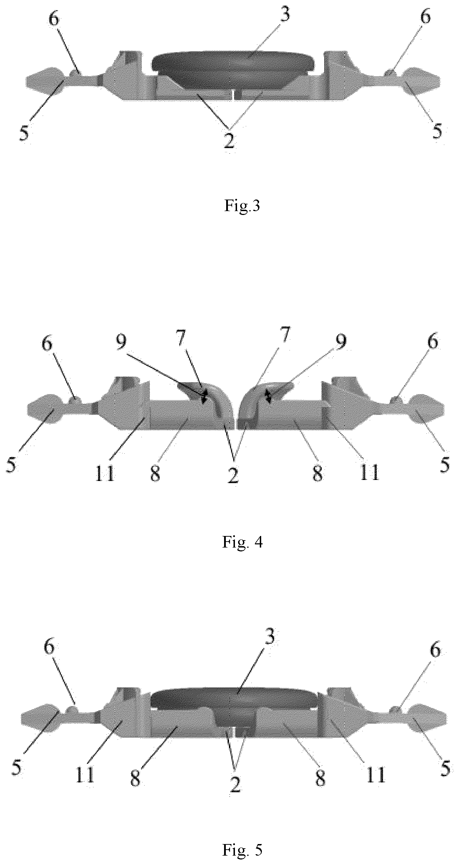

The holder 2 is provided with at least four fasteners for placing the condom 3 therein in a rolled or folded state. The fasteners can be positioned so that the place of their connection to the holder 2 base is on the inside of the condom 3 ring - inner fasteners 7 (Fig.2, 3), or so that the place of their connection to the holder 2 base is on the outside of the condom 3 ring - outer fasteners 8 (Fig.4, 5). The minimum number of fasteners required is four inner ones.

The fasteners 7, 8 are made curved (hook-shaped) to securely hold the condom 3 ring. The diameter of the fasteners 7, 8 bend may be slightly larger or equal to the thickness of the rolled condom 3 ring, but cannot be much smaller, since this may damage the condom 3.

The fasteners 7, 8 can be positioned evenly throughout the perimeter of the condom 3 ring. For example, the four inner fasteners 7 can be positioned evenly in a circle inside the condom 3 ring. The outer fasteners 8 can be positioned evenly from the outside of the condom 3 ring. In this case, the outer fasteners 8 can be positioned accordingly to the inner fasteners 7, for example, opposite or shifted.

In each fastener 7, 8 and/or at least between one pair consisting of an inner fastener 7 and an outer fastener 8, a gap 9 of variable size is formed (Fig.2, 4), which, in one position, allows the folded condom 3 to be placed in the fasteners through it, and in the other position it does not allow the completely folded condom 3 to separate from the fasteners. Thus, after placing the folded condom 3, the gap 9 is reduced and prevents the completely folded condom 9 from falling out of the holder 2.

The gap 9 of variable size can be provided due to the fact that the fasteners 7, 8 are designed to be unbent to place the folded condom 3 ring inside and then take its initial geometry. For this purpose, the fasteners 7, 8 can be made of flexible material. Also, the fasteners 7, 8 can be further provided with a means 10 (for example, a cover plate or a protrusion) of reducing the gap 9.

If the fasteners 7, 8 are hooks extending from the holder 2 base, then the gap 9 size can be measured from the base of the fasteners 7, 8 to the upper end thereof or from the holder 2 base to the upper end of the fasteners. In another embodiment of the fasteners 7, 8, when they are an open ring (or another suitable geometry for holding the rolled condom 3 ring inside, for example, an ellipse, etc.), the size of the gap 9 is determined by the size of a break in the contour of this geometry.

The gap 9 of variable size can be provided due to the fact that the fasteners 7, 8 can be configured to open to place the folded condom 3 ring inside and then close, taking the initial position. The fasteners 7, 8 opening can be ensured due to the flexibility of the manufacturing material of the entire fasteners 7, 8 or at the place of their connection to the holder 2 base or the presence of loops at the place of attachment of the fasteners 7, 8 to the holder 2 base, which have fixed positions (open/closed).

Also, the gap 9 can be formed between the inner 7 and the outer fasteners 8.

The outer fasteners 8 can fix their closed position using stoppers 11 located at the edge of the holder 2 (Fig.4, 5). For example, due to the presence of a stopper 11, a folding fastener 8 can fix its closed position by latching on to it, thereby forming the required reduced size of the gap 9 between it and the corresponding inner fastener 7. The number of stoppers 11 corresponds to the number of outer fasteners 8. Engagement can be provided due to the flexibility of the fastener 8 material and the rigidity of the stopper 11 material or vice versa. In addition, grips may be formed on the fastener 8 (displayed by drawing tentatively) for engagement with the stoppers 11.

In particular, a combination of rigid fasteners paired with flexible or folding ones is possible. For example, the inner fasteners 7 are made rigidly, and the outer fasteners 8 positioned accordingly are made opening/closing and thus forming a varying size of the gap 9 between a pair of fasteners 7 and 8.

The size of the gap 9 can be adjusted depending on the intended thickness of the rolled condom 3 ring. The option of separating the condom 3 from the holder 2 is possible only when the condom 3 is completely unrolled, and a possible option is with at least partial unwinding of it. In both options, the fasteners 7, 8 securely hold the condom 3 in the holder 2 until it is used.

To use the condom 3 as intended the packing for a condom is to be opened. To do so, one has to take the side grippers 5 (if any) or the edges of the holder 2 with both hands and to pull them up or down (in vertical plane) resulting in breaking the covering 1 by the opening line 4. Next, to fully split the covering 1 in two parts one has to pull the side grippers 5 (if any) or the edges of the holder 2 apart (in horizontal plane). Herein, due to the condom 3 fixation by the fasteners 7, 8, the condom 3 would also be stretched and the condom 3 ring diameter would increase. This provides an opportunity for putting the condom onto the erect penis (not shown on drawing) in the correct orientation fast and convenient, without touching the condom 3 with fingers. Upon at least partial unwinding of the condom 3 ring, the thickness of the ring will decrease and the condom 3 will be able to emerge from the gap 9 of the fasteners 7, 8, easily separating from the holder 2.

## Claims

1. Packing for a condom comprising a covering with a holder inside, and the holder is equipped with fasteners designed to hold the condom in a folded state and located on the inner and/or outer side of the folded condom, wherein in each fastener and/or between at least one pair consisting of an inner and an outer fastener a gap of variable size is formed.

2. The packing for a condom according to claim 1, **characterized in that** the covering is provided with an opening line.

3. The packing for a condom according to claim 1, **characterized in that** the holder consists of at least two parts, and each part has fasteners.

4. The packing for a condom according to claim 1, **characterized in that** the holder is provided with side grippers located on two opposite sides of the holder and designed to be held with hand fingers when opening the packing.

5. The packing for a condom according to claim 1, **characterized in that** the fastener is made hook-shaped.

6. The packing for a condom according to claim 5, **characterized in that** the fastener is designed to be unbent to place the folded condom ring inside and then taking initial geometry.

7. The packing for a condom according to claim 1, **characterized in that** the fastener is designed to open to place the folded condom ring inside and then close, taking initial position.
